# EUROPÄISCHE PATENTSCHRIFT

(11) **EP 0 946 276 B1**
(45) Veröffentlichungstag und Bekanntmachung des Hinweises auf die Patenterteilung: **28.03.2001**
(21) Anmeldenummer: 97948714.7
(22) Anmeldetag: 29.10.1997
(51) Int. Cl.: B01J 8/10, B01J 19/18, C07C 31/32, B01J 8/20, B01J 8/00

(54) **VORRICHTUNG ZUM INKONTAKTBRINGEN VON FESTKÖRPERN IN FORM VON SCHÜTTFÄHIGEN STÜCKEN MIT FLÜSSIGKEITEN UND GGF. GASEN**
DEVICE FOR BRINGING SOLID BODIES IN THE FORM OF POURABLE PIECES INTO CONTACT WITH LIQUIDS AND POSSIBLY GASES
DISPOSITIF DESTINE A METTRE DES SOLIDES SOUS FORME DE FRAGMENTS COULANTS EN CONTACT AVEC DES LIQUIDES ET EVENTUELLEMENT DES GAZ

(30) Priorität: 05.11.1996 DE 19645527
(43) Veröffentlichungstag der Anmeldung: 06.10.1999
(73) Patentinhaber: RWE-DEA Aktiengesellschaft für Mineraloel und Chemie, 22297 Hamburg (DE)
(72) Erfinder: ALBERT, Gert, D-25541 Brunsbüttel (DE)
(74) Vertreter: Schupfner, Gerhard D., Dr. Dipl.-Chem.
(86) Internationale Anmeldenummer: DE9702564
(87) Internationale Veröffentlichungsnummer: WO9819785

(56) Entgegenhaltungen:
- EP-A- 0 102 615
- EP-A- 0 111 115
- CH-A- 396 902
- DE-A- 4 439 860

## Beschreibung

Die Erfindung bezieht sich auf eine Vorrichtung zum Inkontaktbringen von Festkörpern in Form von schüttfähigen Stücken mit Flüssigkeiten und ggf. Gasen zum Auflösen der Festkörpern mit einem Gehäuse, in dem eine mit Löchern versehene Wanne angeordnet ist, die die Festkörper aufnimmt und von der Flüssigkeit umspült als Sprudelbett ausgebildet ist.

Aus der DE 32 44 972 C1 ist eine solche Vorrichtung bekannt. Diese besteht aus einem Reaktorgehäuse und einer in dem Reaktorgehäuse angeordneten Wanne, in den Aluminiumstücke in Form von Nadeln oder Spänen als Partikel eingebracht sind. Ein Aluminiumalkoholat/Alkohol-Gemisch wird so sprudelnd in Wallung gebracht, daß es das fein zerteilte Aluminium in dem Siebkorb aufwirbelt. Bei diesem Umspülen löst sich das Aluminium im Aluminiumalkoholat/Alkohol-Gemisch auf.

Es ist Aufgabe der Erfindung, das Umsatzvolumen des Reaktors zu erhöhen und den Einsatz von großvolumigen Festkörper-Partikel, vorzugsweise Barrenabschnitten, zu ermöglichen.

Die gestellte Aufgabe ist erfindungsgemäß dadurch gelöst, daß ein Drehrost vorgesehen ist zur zumindest zeitweisen Aufnahme der Festkörper.

Mit diesem Drehrost läßt sich das Umsatzvolumen deutlich verbessern. Die verarbeitbare Partikelgröße der Festkörper ist so erhöht, daß auch Barrenabschnitte von mehr als 1 kg Gewicht verarbeitbar sind, was zuvor nicht möglich war.

Nach einer weiteren Ausgestaltung der Erfindung ist vorgesehen, daß zum Einbringen der Festkörper in den Drehrost ein Fallrohr vorgesehen ist, mit dem die Festkörper von oben gleichmäßig auf die Fläche des Siebreaktors verteilbar ist. Dazu ist nach einer weiteren Ausgestaltung des Erfindung vorgesehen, daß das Fallrohr derart an einem Gelenk angeordnet ist, daß seine unten gelegene Auslaßöffnung zwischen dem Außenbereich des Drehrostes und dessen Mitte verstellbar und daß das Fallrohr mittels einer von außen bedienbaren Stellvorrichtung verstellbar ist.

Nach einer weiteren Ausgestaltung der Erfindung ist vorgesehen, daß der korbartig ausgebildete Drehrost an einer vertikalen und zentralen Welle angeordnet ist, die an der Reaktoroberseite abgedichtet aus dem Reaktor herausgeführt ist und außerhalb des Reaktors mittels eines Motors drehbar ist.

Nach einer weiteren Ausgestaltung der Erfindung ist dazu vorgesehen, daß die Drehgeschwindigkeit des Drehrostes so einstellbar ist, daß die in den Drehrost eingebrachten Festkörper innerhalb einer Drehrostumdrehung aufgelöst sind.

Nach einer weiteren Ausgestaltung der Erfindung ist vorgesehen, daß der äußere Rand des Drehrostes auf einer Konsole, die an der Innenwand des Reaktors angeordnet ist, drehbar abgestützt ist. Der Drehrost ist auf diese Weise gut geführt.

Nach einer weiteren Ausgestaltung der Erfindung ist vorgesehen, daß um den Drehrost herum auf den Siebkorb gerichtete Reinigungsdüsen angeordnet sind, aus denen eine Flüssigkeit oder ein Flüssigkeitsgemisch auf den Siebkorb spritzbar ist. Damit wird Verstopfungen der Siebkorblöcher vorgebeugt.

Nach einer weiteren Ausgestaltung der Erfindung ist vorgesehen, daß die Reinigungsdüsen an einer Rohrleitung vorgesehen sind, die durch die hohle Welle des Drehrostes in einem Zwischenraum zwischen dem Siebkorb und dem Drehrost geführt und von dort nach außen geführt ist.

Nach einer weiteren Ausgestaltung der Erfindung ist vorgesehen, daß die Rohrleitung im Zwischenraum vom äußeren Ende eines radial geführten Rohrleitungsteiles in einen nach oben abgebogenen Rohrleitungsteil übergeht.

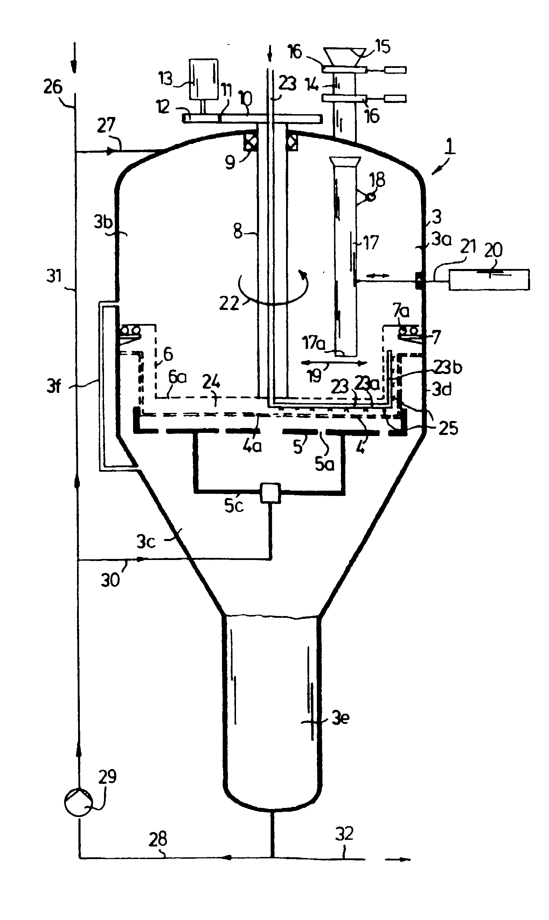

Die Erfindung wird anhand der Zeichnung näher erläutert.

Die Figur zeigt einen Reaktor zum Herstellen eines Aluminiumalkoholat/Alkohol-Gemisches mit einem Siebkorb und einem darüber befindlichen Drehrost.

Die Zeichnung zeigt einen Reaktor 1 mit einem Reaktorgehäuse 3. Im Inneren 3a des Reaktorgehäuses 3 befindet sich ein Siebkorb 4, der ein Oberteil 3b des Reaktors von einem Unterteil 3c trennt. Es ist eine Verbindungsleitung 3f vorgesehen, die einen Druckausgleich zwischen dem unterenTeil 3c des Reaktors 1 und dessen oberem Teil 3b herbeiführt. Diese Verbindungsleitung 3f ist wichtig, da anderenfalls infolge von Reaktionsgas- und Dampfbildung im Sumpf Unregelmäßigkeiten in dem Siebkorb 4 auftreten können. Der Siebkorb 4 ist mit Löchern 4a versehen und auf nicht näher dargestellte Weise an der Wand 3d des Reaktorgehäuses 3 befestigt. Unterhalb des Siebkorbs 4 befindet sich eine Wanne 5, die ebenfalls mit Durchbrechungen 5a versehen ist. In die Wanne 5 führen mehrere Leitungen 5c, die beim Betrieb ein Aluminiumalkoholat/Alkohol-Gemisch in die Wanne 5 einleiten, das auf noch zu beschreibende Weise über die eingefüllten Aluminiumpartikel sprudelt und damit ein Sprudelbett bildet. Oberhalb des Siebkorbes 4 befindet sich ein Drehrost 6. Dieser Drehrost 6 ist ebenfalls gelocht. Dies ist durch Unterbrechungen 6a angedeutet. Der Drehrost 6 ruht auf Lagern 7a einer Konsole 7.

Der Drehrost 6 ist an einer Hohlwelle 8 befestigt, die über ein Lager 9 mit Stopfbuchs-und Gleitringdichtung zu einem Zahnrad eines Getriebes 11 geführt ist. Das Zahnrad 10 des Getriebes 11 kämmt mit einem Zahnrad 12, das von einem Motors 13 antreibar ist.

Es ist eine nicht näher beschriebene Aufgabeschleuse 14 vorgesehen, durch die über einen Trichter 15 grobe Aluminiumpartikel, vorzugsweise Barrenabschnitte auch mit mehr als 1 kg Gewicht, in den Reaktor einfüllbar sind. Bei wechselnd geöffneten Schiebern 16 fallen diese Partikel durch die Aufgabeschleuse 14 in ein Fallrohr 17. Dieses Fallrohr 17 ist an einem Lager 18 angeordnet, welches das Schwenken der unteren Auslaßöffnung 17a des Fallrohres 17 in Richtung eines Doppelpfeiles 19 vom Inneren des Drehrostes 6 zu seinem äußeren Bereich ermöglicht. Das Verstellen oder Verschwenken des Fallrohres 17 erfolgt über einen Stelltrieb 20 auf der Außenseite des Reaktors, der mittels einer Stellstange 21 mit dem Fallrohr 17 verbunden ist.

Durch die Welle 8 des Drehrostes 6, der in Richtung eines Pfeiles 22 drehbar ist, führt eine Rohrleitung 23 in einen Zwischenraum 24 zwischen dem Drehrost 6 und dem Siebkorb 4. Die Rohrleitung 23 dreht sich zusammen mit der Welle 8. In dem Zwischenraum 24 ist die Rohrleitung nach außen und nach oben geführt. An dem nach außen geführten Teil 23a und an dem vom äußeren Ende des Teiles 23a nach oben geführten Teil 23b der Leitung 23 befinden sich Reinigungsdüsen 25, die auf den Siebkorb 4 gerichtet sind.

Über einen Leitungsabschnitt 26 kann frisches Hexanol dem Reaktor 1 zugeführt werden. Dieses frische Hexanol strömt über eine Leitung 27 in den Innenraum 3a des Reaktorgehäuses ein. Über das Fallrohr 17 wurden zuvor Aluminiumpartikel auf den Drehrost 6 aufgegeben. Die aufgegebenen Aluminiumpartikel sind sehr grob und werden vorzugsweise Barrenabschnitte sein. Der Drehrost 6 hat einen Abstand von 5 bis 10 cm von dem Siebkorb 4. Der Antrieb 11, 13 des Drehrostes 6 ist so ausgelegt, daß die durch das Fallrohr 17 gleichmäßig auf den Drehrost 6 aufgegebenen Partikel bei einer Rostumdrehung aufgelöst sind. Es ist dabei wichtig, daß über das Fallrohr 17 auf den Drehrost 6 eine Schicht von Aluminiumpartikeln aufgegeben wird, die beispielsweise 10-15 cm dick ist .

Nach der Inbetriebnahme des Reaktors sammelt sich im Sumpf 3e des Reaktorgehäuses 3 ein Aluminiumalkoholat/Alkohol-Gemisch . Dieses kann über eine Leitung 28 und eine Pumpe 29 sowie eine Leitung 30 der Wanne 5 zugeleitet werden. Das Einführen des Aluminiumalkoholat/Alkohol-Gemisches erfolgt mit so hohem Druck, daß eine wallende Sprudelwirkung und somit ein Sprudelbett erzielt wird. Die sprudelnde Flüssigkeit sprudelt durch den Siebkorb 4 und den Drehrost 6 über alle im Reaktor befindlichen Aluminiumpartikel. Das Aluminiumalkoholat/Alkohol-Gemisch strömt zusätzlich über eine Leitung 31 und die Leitung 27 im oberen Bereich 3b des Reaktorgehäuses 3.

Wird die Pumpe 29 abgeschaltet, dann hört das Sprudeln der Flüssigkeit unverzüglich auf. Die Flüssigkeit fällt zurück in den Sumpf 3e und die Berührung der Flüssigkeit mit den Aluminiumpartikeln hört auf. Damit ist auch die Reaktion praktisch schlagartig unterbrochen.

Ein Teil des Aluminiumalkoholat/Alkohol-Gemisches kann über eine Leitung 32 abgeführt werden.

Die Vorrichtung hat ein außerordentlich großes Durchsatzvolumen. Auf die beschriebene Weise sind vorhandene Reaktoren gemäß der DE 32 44 972 Cl ohne Schwierigkeiten für größeren Umsatz und größere Partikel umrüstbar.

## Patentansprüche

1. Vorrichtung zum Inkontaktbringen von Festkörpern in Form von schüttfähigen Stücken mit Flüssigkeiten und ggf. Gasen zum Auflösen der Festkörper mit einem Gehäuse (3), in dem eine mit Löchern versehene Wanne (5) angeordnet ist, die die Festkörper aufnimmt und von der Flüssigkeit umspült als Sprudelbett ausgebildet ist, dadurch gekennzeichnet, daß ein Drehrost (6) vorgesehen ist zur zumindest zeitweisen Aufnahme der Festkörper.

2. Vorrichtung nach Anspruch 1, dadurch gekennzeichnet, daß der Drehrost (6) oberhalb eines Siebkorbes (4) vorgesehen ist.

3. Vorrichtung nach Anspruch 1 und/oder 2, dadurch gekennzeichnet, daß zum Einbringen der Festkörper in den Drehrost (6) ein Fallrohr (17) vorgesehen ist, mit dem die Festkörper von oben gleichmäßig auf die Reaktorfläche verteilbar sind.

4. Vorrichtung nach Anspruch 3, dadurch gekennzeichnet, daß das Fallrohr (17) derart an einem Gelenk (18) angeordnet ist, das seine unten gelegene Auslaßöffnung (17a) zwischen dem Außenbereich des Drehrostes (6) und dessen Mitte verstellbar ist.

5. Vorrichtung nach Anspruch 3 und/oder 4, dadurch gekennzeichnet, daß das Fallrohr (17) mittels einer von außen bedienbaren Stellvorrichtung (20) verstellbar ist.

6. Vorrichtung nach einem oder mehreren der Ansprüche 1 bis 3, dadurch gekennzeichnet, daß der Drehrost (6) korbartig ausgebildet und an einer vertikalen und zentralen Welle (8) angeordnet ist.

7. Vorrichtung nach Anspruch 6, dadurch gekennzeichnet, daß die Welle (8) an der Reaktoroberseite abgedichtet aus dem Reaktor (1) herausgeführt ist und außerhalb des Reaktors mittels eines Motors (13) drehbar ist.

8. Vorrichtung nach Anspruch 7, dadurch gekennzeichnet, daß die Drehgeschwindigkeit des Drehrostes (6) so einstellbar ist, daß die in den Drehrost (6) eingebrachten Aluminiumstücke innerhalb einer Drehrostumdrehung aufgelöst sind.

9. Vorrichtung nach einem oder mehreren der Ansprüche 1 bis 8, dadurch gekennzeichnet, daß der äußere Rand des Drehrostes (6) auf einer Konsole (7), die an der Innenwand des Reaktors angeordnet ist, drehbar abgestützt ist.

10. Vorrichtung nach einem oder mehreren der Ansprüche 1 bis 9, dadurch gekennzeichnet, daß um den Drehrost (6) herum auf den Siebkorb (4) gerichtete Reinigungsdüsen (25) angeordnet sind.

11. Vorrichtung nach Anspruch 10, dadurch gekennzeichnet, daß die Reinigungsdüsen (25) an einer Rohrleitung (23) vorgesehen sind, die durch die hohle Welle (8) des Drehrostes (6) in einem Zwischenraum (24) zwischen dem Siebkorb (4) und dem Drehrost (6) geführt und von dort nach außen geführt ist.

12. Vorrichtung nach Anspruch 11, dadurch gekennzeichnet, daß die Rohrleitung (23) im Zwischenraum (24) vom äußeren Ende eines radial geführten Rohrleitungsteiles (23a) in einen nach oben abgebogenen Rohrleitungsteil (23b) übergeht.

13. Vorrichtung nach einem der vorhergehenden Ansprüche zur kontinuierlichen Herstellung von Aluminiumalkoholaten durch Umsetzen von Aluminium mit einem Überschuß von aliphatischem C₃- bis C₁₀ -Alkohol.

## Claims

1. An apparatus for contacting solids in the form of flowable lumps with liquids and, optionally, gases in order to dissolve said solids using a housing (3) wherein a perforated vat (5) holding the solids is disposed which, as the liquid flows around, forms a spouted bed,
characterized in that there is provided a rotary grate (6) for holding the solids at least temporarily.

2. An apparatus according to claim 1,
characterized in that said rotary grate (6) is disposed above a screen basket (4).

3. An apparatus according to claim 1 and/or 2,
characterized in that there is provided a vertical pipe (17) for placing the solids into the rotary grate (6), said pipe allowing to charge the solids from the top and to distribute them evenly in the reaction space.

4. An apparatus according to claim 3,
characterized in that said vertical pipe (17) is provided with a joint (18) such that the lower pipe outlet (17a) can be shifted between edge and center of the rotary grate (6).

5. An apparatus according to claim 3 and/or 4,
characterized in that said pipe (17) is adjustable by means of a device (20) which is steerable from outside.

6. An apparatus according to any one or a plurality of claims 1 to 3,
characterized in that the rotary grate (6) has the shape of a basket and is disposed on a central, vertical shaft (8).

7. An apparatus according to claim 6, characterized in that said shaft (8) protrudes from the top of the reactor (1), the protrusion point being sealed and the shaft being rotatable from outside the reactor using a motor (13).

8. An apparatus according to claim 7,
characterized in that the rotational speed of the rotary grate (6) is adjustable such that the aluminium lumps placed therein will be dissolved after a single turn of the grate (6).

9. An apparatus according to any one or a plurality of claims 1 to 8,
characterized in that the edge of the rotary grate (6) is supported by a console (7) mounted on the interior wall of the reactor, the rotary grate thus remaining rotatable.

10. An apparatus according to any one or a plurality of claims 1 to 9,
characterized in that purifying jets (25) arranged around the rotary grate (6) arc directed at the screen basket (4).

11. An apparatus according to claim 10,
characterized in that the purifying jets (25) are arranged on a pipe (23) which is led through the hollow shaft (8) of the rotary grate (6) into a space (24) between screen basket (4) and rotary grate (6) and extends there-from to the outside.

12. An apparatus according to claim 11,
characterized in that the pipe (23) extends in the space (24) from the outer end of a radial pipe section (23a) to an upward-bent pipe section (23b).

13. An apparatus according to any one of the preceding claims for the continuous production of aluminium alcoholates by reacting aluminium with an excess of aliphatic C₃ to C₁₀ alcohol.

## Revendications

1. Dispositif pour mettre des corps solides sous la forme de pièces pouvant être mis en vrac, en contact avec des liquides et éventuellement des gaz pour dissoudre les corps solides, comprenant une enveloppe (3) dans laquelle est agencé un bac (5) pourvu de trous, qui reçoit les corps solides et arrosé par le liquide, se présente sous la forme d'un lit fluidisé par jaillissement, caractérisé en ce qu'il est prévu une grille tournante (6) pour recevoir les corps solides au moins temporairement.

2. Dispositif selon la revendication 1, caractérisé en ce que la grille tournante (6) est disposée au-dessus d'un panier de criblage (4).

3. Dispositif selon la revendication 1 et/ou 2, caractérisé en ce qu'il est prévu, pour introduire les corps solides dans la grille tournante (6), un tube de descente (17) par lequel les corps solides peuvent être distribués sur la surface du réacteur de manière uniforme, par le dessus.

4. Dispositif selon la revendication 3, caractérisé en ce que le tube de descente (17) est agencé sur une articulation (18) de telle sorte que son ouverture de sortie inférieure (17a) puisse être réglée entre la zone externe de la grille tournante (6) et son centre.

5. Dispositif selon la revendication 3 et/ou 4, caractérisé en ce que le tube de descente (17) peut être réglé au moyen d'un dispositif de réglage (20) qui peut être commandé de l'extérieur.

6. Dispositif selon une ou plusieurs des revendications 1 à 3, caractérisé en ce que la grille tournante (6) est conformée en panier et est agencée sur un arbre vertical et central (8).

7. Dispositif selon la revendication 6, caractérisé en ce que l'arbre (8) est guidé hors du réacteur (1) de manière étanche sur la partie supérieure du réacteur et peut être soumis à une rotation à l'extérieur du réacteur au moyen d'un moteur (13).

8. Dispositif selon la revendication 7, caractérisé en ce que la vitesse de rotation de la grille tournante (6) peut être réglée de telle sorte que les pièces d'aluminium introduits dans la grille tournante (6) soient dissous en l'espace d'une révolution de la grille tournante.

9. Dispositif selon une ou plusieurs des revendications 1 à 8, caractérisé en ce que le bord extérieur de la grille tournante (6) est monté à rotation sur une console (7) qui est agencée sur la paroi interne du réacteur.

10. Dispositif selon une ou plusieurs des revendications 1 à 9, caractérisé en ce que des buses de nettoyage (25) dirigées vers le panier de criblage (4) sont agencées autour de la grille tournante (6).

11. Dispositif selon la revendication 10, caractérisé en ce que les buses de nettoyage- (25) sont disposées sur une conduite tubulaire (23), qui est guidée à travers l'arbre creux (8) de la grille tournante (6) dans un espace intermédiaire (24) compris entre le panier de criblage (4) et la grille tournante (6) et de là vers l'extérieur.

12. Dispositif selon la revendication 11, caractérisé en ce que la conduite tubulaire (23) se fond dans l'espace intermédiaire (24) d'une extrémité extérieure d'une partie de conduite tubulaire guidée radialement (23a) en une partie de conduite tubulaire (23b) coudée vers le haut.

13. Dispositif selon l'une quelconque des revendications précédentes pour la préparation en continu d'alcoolates d'aluminium par transformation d'aluminium avec un excès d'alcool aliphatique en C₃-C₁₀.
